(19) Europäisches Patentamt
European Patent Office
Office européen des brevets

(11) EP 4 810 477 A1

(12) EUROPEAN PATENT APPLICATION
published in accordance with Art. 153(4) EPC

(43) Date of publication:
**23.09.2026 Bulletin 2026/39**

(21) Application number: **24890357.7**

(22) Date of filing: **29.09.2024**

(51) International Patent Classification (IPC):
***C07C 67/08*** *(2006.01)*

(52) Cooperative Patent Classification (CPC):
**C07C 29/80; C07C 31/20; C07C 67/08; C07C 67/54; C07C 69/675**

(86) International application number:
**PCT/CN2024/122229**

(87) International publication number:
**WO 2025/102999 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.11.2023 CN 202311520352**

(71) Applicants:
- **China Petroleum & Chemical Corporation**
  **Beijing 100728 (CN)**
- **Sinopec (Beijing) Research Institute of Chemical Industry Co., Ltd.**
  **Beijing 100013 (CN)**

(72) Inventors:
- **SHI, Qian**
  **Beijing 100013 (CN)**
- **GUO, Liang**
  **Beijing 100013 (CN)**
- **LUO, Shujuan**
  **Beijing 100013 (CN)**
- **ZHU, Yuehui**
  **Beijing 100013 (CN)**
- **LI, Yan**
  **Beijing 100013 (CN)**
- **TIAN, Jun**
  **Beijing 100013 (CN)**
- **SHAO, Huawei**
  **Beijing 100013 (CN)**
- **GAO, Jinglin**
  **Beijing 100013 (CN)**
- **FAN, Xiaozhe**
  **Beijing 100013 (CN)**
- **CHEN, Guanyu**
  **Beijing 100013 (CN)**

(74) Representative: **karo IP Patentanwälte PartG mbB**
**Steinstraße 16-18**
**40212 Düsseldorf (DE)**

# (54) COMBINED TREATMENT METHOD FOR MIXED ALCOHOL WASTE AND HEAVY ALCOHOL WASTE

(57) The present invention relates to a joint treatment method for a mixed alcohol waste and a heavy alcohol waste, characterized in that said joint treatment method comprises the following reaction steps:
Step 1. optionally, subjecting the mixed alcohol waste primarily comprising C1-C4 saturated monohydric alcohols to preliminary separation treatment to at least partially remove contained water and acid, and optionally at least partially separating out a heavy component primarily comprising esters, to obtain a preliminary purification product of said mixed alcohol waste;
Step 2. mixing the mixed alcohol waste that optionally has been preliminarily separated, said heavy alcohol waste, and optionally added alcohol, and allowing them to react in the presence of an optional catalyst, to obtain a product containing glycolates and ethylene glycol; and, optionally
Step 3. separating a material containing glycolates and ethylene glycol from the product obtained in Step 2 and optionally from the heavy component obtained in Step 1; wherein said heavy alcohol waste comprises at least one of ethylene glycol, glycolic acid, and methyl glycolate, and polymers produced by polymerization reaction of at least one of these compounds.

The present invention also relates to a system for implementing the method according to the present invention.

EP 4 810 477 A1

mixed alcohol waste
preliminary separation unit
first intermediate component
first light component
second light component
first heavy component
heavy alcohol waste
reaction unit
product separation unit
methanol
second intermediate component
second heavy component

FIG.1

## Description

### Technical Field

The present invention relates to the field of chemical waste treatment. Specifically, the present invention relates to a joint treatment method for a mixed alcohol waste and a heavy alcohol waste.

### Background Art

Polyglycolic acid is the polymer with the fastest biodegradable rate among aliphatic thermoplastic linear polyesters. Due to its high heat resistance, high strength, fast degradation rate, strong oxygen barrier properties, strong solvent resistance, excellent performance, and certain processability allowing processing by conventional equipment such as extrusion, injection molding, spinning, and blow molding, it is widely used in many fields such as medical, agricultural/forestry, and packaging. It is a key new area of development in developed countries like Japan and the USA.

In recent years, with the breakthrough of coal-to-ethylene glycol technology in China, the industrialization of PGA is imminent. The capacity of polyglycolic acid plants under construction or planned in China is about 1.13 million tons. This production route generates a large amount of fusel oil as by-product, wherein organic compounds account for more than 50% of the waste, mainly monohydric alcohols, dihydric alcohols, etc. Due to the numerous impurity components in these fusel wastes, the close boiling points of the various substances, and the existence of some as azeotropes, the direct separation of the components is difficult, therefore, currently, these by-products are sold as waste at a price of 500-800 RMB/ton. These high-value-added alcohol substances are sold at low prices as waste, causing great waste of enterprise resources, which does not comply with national circular economy industrial policies. Furthermore, the sold waste is often subjected to only simple separation by many small enterprises, resulting in low utilization rates, high energy consumption, and the remainder is discharged as wastewater, causing environmental pollution. However, to date, there has been no disclosure on how to simply and effectively recover and treat these fusel wastes.

CN108250481A discloses a method for catalytic alcoholysis of waste PET, using homogeneous catalysts such as tungstate and zinc acetate, with dihydric alcohols as solvents. After alcoholysis, residual catalysts result in insufficient purity of the products, leading to lower hydroxyl values of the depolymerization products, and the excessive content of heavy metal ions in the catalyst adversely affects the repolymerization and subsequent processing applications of the products.

CN111203202A discloses a polymer catalyst degradation method for treating polymer materials such as polyethers, polyesters, polysiloxanes, polyurethanes, and polyamides, but similarly uses metal compounds containing active hydroxyl groups on the surface as homogeneous catalysts, requiring consideration of the impact on subsequent processing.

CN114031600A discloses a method for recovering caprolactone from poly-ε-caprolactone waste, which can be performed under solvent-free conditions but requires vacuum conditions, making the conditions more stringent.

Therefore, there is an urgent need for a method capable of treating fusel oil (including mixed alcohol waste and heavy alcohol waste) derived from polyglycolic acid production plants or from oxalate process for producing ethylene glycol or methyl glycolate, to recover high-value-added components such as methyl glycolate and ethylene glycol, and this method should feature simple steps, fast reaction rate, and no generation of other complex by-products.

### Summary of the Invention

The object of the present invention is to overcome the problems in the prior art where processes for treating fusel oil (including mixed alcohol waste and heavy alcohol waste) derived from polyglycolic acid preparation plants are complex and the generated by-products affect the subsequent processing of the desired products. The present invention provides a joint treatment method for a mixed alcohol waste and a heavy alcohol waste, this method can fully utilize the mixed alcohol waste and heavy alcohol waste, recover high-value-added components such as methyl glycolate, without generating complex by-products and without affecting the quality of subsequent products.

To achieve the above object, after in-depth research, the inventors surprisingly found that if the mixed alcohol waste and heavy alcohol waste from polyglycolic acid preparation process or other processes are jointly treated to allow their main components to react (such as alcoholysis), to can simply and effectively recover high-value target compounds, mainly glycolates and ethylene glycol. This reaction system has low purity requirements for the alcohol; the alcohol can contain low-boiling ester impurities at a content less than or equal to 20 wt%, and such impurity content does not affect the yield and purity of the glycolates. Furthermore, the inventors surprisingly found that, compared to methods treating the mixed alcohol waste and heavy alcohol waste separately, the method of the present invention can significantly increase the yield of these high-value target compounds and reduce separation and purification operations.

Therefore, according to a first aspect of the present invention, the present invention provides a joint treatment

method for a mixed alcohol waste and a heavy alcohol waste, characterized in that said joint treatment method comprises the following reaction steps:

Step 1. optionally, subjecting the mixed alcohol waste primarily comprising C1-C4 saturated monohydric alcohols to preliminary separation treatment to at least partially remove contained water and acid, and optionally at least partially separating out a heavy component primarily comprising esters (e.g., glycolates and polymers thereof), to obtain a preliminary purification product of said mixed alcohol waste;

Step 2. mixing the mixed alcohol waste that optionally has been preliminarily separated, said heavy alcohol waste, and optionally added alcohol, and allowing them to react in the presence of an optional catalyst, to obtain a product containing glycolates and ethylene glycol; and optionally

Step 3. a step of separating a material containing glycolates and ethylene glycol from the product obtained in Step 2 and optionally from the heavy component obtained in Step 1;

wherein said heavy alcohol waste comprises at least one of ethylene glycol, glycolic acid, and methyl glycolate, and polymers produced by polymerization reaction of at least one of these compounds.

According to a second aspect of the present invention, the invention provides a system for implementing a joint treatment method for a mixed alcohol waste and a heavy alcohol waste according to the first aspect described above, comprising: an optional preliminary separation unit, a reaction unit, and a product separation unit, wherein said preliminary separation unit is used to at least partially remove water and acid contained in the mixed alcohol waste and optionally separate out a heavy component primarily comprising esters to obtain a preliminary purification product of the mixed alcohol waste; said reaction unit is used to mix heavy alcohol waste, added alcohol, the preliminary purification product of the mixed alcohol waste, and optionally the heavy component primarily comprising esters, and allow them to react in the presence of an optional catalyst; and said product separation unit is used to separate a material containing glycolates and ethylene glycol from the reaction product coming from said reaction unit.

The joint treatment method and the system for implementing said method according to the present invention have at least the following beneficial effects:
the joint treatment method for a mixed alcohol waste and a heavy alcohol waste according to the present invention has simple process steps, fast reaction rate, no generation of complex by-products, no impact on the quality of the desired product, good economic practicality, can fully utilize the main components in mixed alcohol waste and heavy alcohol waste, significantly improve the yield of useful products, save the separation steps of the mixed alcohol waste and heavy alcohol waste respectively, thereby reducing energy consumption, and recover high-value-added components such as materials containing glycolates, thus achieving quality improvement and efficiency enhancement of mixed alcohol waste and heavy alcohol waste.

In the method according to the present invention, organic compounds in fusel oil (including mixed alcohol waste and/or heavy alcohol waste) generated during polyglycolic acid production are recovered to obtain high-value-added components such as methyl glycolate and ethylene glycol, turning waste into valuable resources, improving the quality and benefits of PGA by-products, enabling maximization of enterprise economic benefits while meeting green production requirements. Additionally, by selecting appropriate preliminary separation methods and conditions for the mixed alcohol waste, selecting appropriate types of added alcohol, introducing suitable types and amounts of catalysts, selecting appropriate reaction feedstock ratios, reaction conditions, and suitable separation conditions, the yield and purity of the material containing glycolates can be further improved.

Brief Description of the Figures

FIG. 1 shows a flowchart of a joint treatment method for a mixed alcohol waste and a heavy alcohol waste according to one embodiment of the present invention;

FIG. 2 shows a flowchart of a joint treatment method for a mixed alcohol waste and a heavy alcohol waste according to another embodiment of the present invention.

Detailed Description of The Invention

The specific embodiments of the present application are described in detail below with reference to the

accompanying drawings. It should be understood that the specific embodiments described herein are only for illustrating and explaining the present invention and are not intended to limit the present invention.

Any specific numerical values disclosed in this specification (including endpoints of numerical ranges) are not limited to the precise values, but should be understood to also cover values close to these precise values, such as all possible numerical values within the range of the precise value $\pm$5%. Furthermore, for the disclosed numerical ranges, one or more new numerical ranges can be obtained by arbitrary combination between the endpoints of the range, between the endpoints and specific point values within the range, and between the specific point values. These new numerical ranges should also be considered as specifically disclosed in this specification.

Unless otherwise stated, the terms used in this specification have the same meanings as those usually understood by those skilled in the art. If a term is defined in this specification and its definition differs from the usual understanding in the art, the definition in this specification shall prevail.

In this specification, except for matters explicitly stated, any unmentioned matter or item directly applies to those known in the art without any change. Moreover, any embodiment described in this specification can be freely combined with one or more other embodiments described herein, and the technical solutions or technical concepts formed thereby are considered as a part of the original disclosure or original description of the present invention and should not be considered as new content not previously disclosed or anticipated in this specification, unless those skilled in the art deem such combination obviously unreasonable.

Unless otherwise explicitly stated, throughout the specification and claims, the term "comprise" or variations such as "include" or "contain" shall be understood as including the stated elements or components, but not excluding other elements or components.

In this specification, terms "first", "second", etc., are used to distinguish two different elements or components, and are not used to limit specific positions or relative relationships. In other words, in some embodiments, terms "first", "second", etc., can also be interchanged. The expression "primarily comprising" indicates that the component accounts for a higher weight or molar proportion of the composition than other components. In this specification, the term "alcohol" generally refers to monoalcohols, excluding dihydric alcohols, unless specifically stated otherwise.

According to the first aspect of the present invention, the present invention provides a joint treatment method for a mixed alcohol waste and a heavy alcohol waste, characterized in that said joint treatment method comprises the following reaction steps:

Step 1: optionally, subjecting the mixed alcohol waste primarily comprising C1-C4 saturated monohydric alcohols to preliminary separation treatment to at least partially remove contained water and acid, and optionally at least partially separating out a heavy component primarily comprising esters (e.g., glycolates and polymers thereof), to obtain a preliminary purification product of said mixed alcohol waste;

Step 2: mixing said mixed alcohol waste that optionally has been preliminarily separated, said heavy alcohol waste, and optionally added alcohol, and allowing them to react in the presence of an optional catalyst, to obtain a product containing glycolates and ethylene glycol; and optionally

Step 3: separating a material containing glycolates and ethylene glycol from the product obtained in Step 2 and optionally from the heavy component obtained in Step 1;

wherein said heavy alcohol waste comprises at least one of ethylene glycol, glycolic acid, and methyl glycolate, and polymers produced by polymerization reaction of at least one of these compounds.

In the above reaction Step 2, other possible substances can also be added, provided that said substances do not negatively affect the reaction occurring in the reaction step, the properties of the desired product, and subsequent processing.

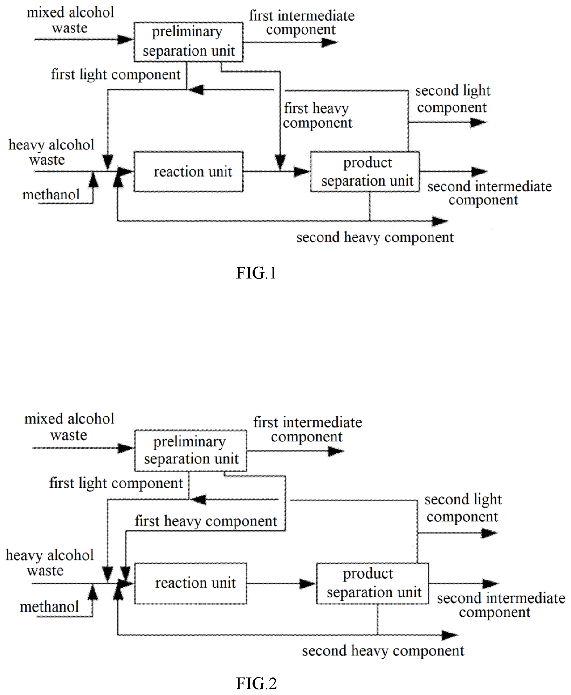

According to one specific embodiment of the aforementioned first aspect of the present invention, as shown in FIG. 1, the present invention provides a joint treatment method for a mixed alcohol waste and a heavy alcohol waste, said joint treatment method comprising the following steps:

S1. optionally, subjecting said mixed alcohol waste to preliminary separation to at least partially separate out a heavy component primarily comprising esters, referred to as "first heavy component", and at least partially remove water and acid contained in said mixed alcohol waste, referred to as "first intermediate component", to obtain a preliminary purification product of said mixed alcohol waste, referred to as "first light component";

S2. mixing said heavy alcohol waste, optionally added alcohol, and either said mixed alcohol waste not subjected to preliminary separation treatment (if step S1 is not performed) or said preliminary purification product of the mixed

alcohol waste obtained in step S1 (if step S1 is performed), in the presence of an optional catalyst, to conduct reaction; and

S3. combining the reaction product obtained in step S2 and the first heavy component obtained in step S1, and separating therefrom a material containing glycolates and ethylene glycol.

According to another specific embodiment of the aforementioned first aspect of the present invention, as shown in FIG. 2, the present invention provides a joint treatment method for a mixed alcohol waste and a heavy alcohol waste, said joint treatment method comprising the following steps:

S1. optionally, subjecting said mixed alcohol waste to preliminary separation treatment to at least partially separate out a heavy component primarily comprising esters, referred to as "first heavy component", and at least partially remove water and acid contained in said mixed alcohol waste, referred to as "first intermediate component", to obtain a preliminary purification product of said mixed alcohol waste, referred to as "first light component";

S2. mixing said heavy alcohol waste, optionally added alcohol, and either said mixed alcohol waste not subjected to preliminary separation treatment (if step S1 is not performed) or said preliminary purification product of the mixed alcohol waste and said first heavy component obtained in step S1 (if step S1 is performed), in the presence of an optional catalyst, to conduct reaction; and

S3. separating a material containing glycolates and ethylene glycol from the reaction product obtained in step S2.

In the joint treatment method according to the first aspect of the present invention, whether to perform the preliminary separation step on said mixed alcohol waste mainly depends on the amount of water and acid contained in the mixed alcohol waste. Since water and acid contained in the mixed alcohol waste can corrode or poison the catalyst used in the reaction step, affecting the active sites of the catalyst and causing easy deactivation of the catalyst, advantageously, before participating in said reaction step, the water content in the mixed alcohol waste is made to be lower than 4%, advantageously lower than 3%, even lower than 1%; simultaneously, the acid content in the mixed alcohol waste participating in said reaction step is lower than 3%, advantageously lower than 2%, even lower than 1%. Most preferably, said mixed alcohol waste participating in said reaction step contains almost no water or acid. If the mixed alcohol waste participating in the reaction step itself contains sufficiently low levels of water and acid, such as less than 1% water and less than 0.5% acid, the preliminary separation step or step S1 of the mixed alcohol waste can be omitted, provided it does not significantly affect the catalyst activity. An additional advantage of performing preliminary separation on the mixed alcohol waste is the removal of low-boiling ester, ketone, and ether impurities contained in the mixed alcohol waste, as these impurities may interfere with subsequent reaction steps.

In the joint treatment method according to the first aspect of the present invention, the mixed alcohol waste is subjected to preliminary separation to respectively obtain a first light component, a first intermediate component, and a first heavy component. The first light component, as the preliminary purification product of the mixed alcohol waste, primarily comprises light alcohols such as C1-C4 alcohols and is fed into the reaction step as a solvent or reactant to participate into the reaction. The first intermediate component primarily comprises impurities such as water, acid, ketones, and ethers and is discarded. The first heavy component primarily comprises ester compounds, especially hydroxypropionate compounds and/or polymers thereof, which are fed into the reaction unit (step) to participate in the reaction or directly fed into the separation unit (step) to be combined with the reaction product obtained in the reaction unit and then subjected to separation together. The separated material containing glycolates and optionally ethylene glycol is recovered as product.

According to one embodiment of the aforementioned first aspect, said mixed alcohol waste can be a by-product from a polyglycolic acid production plant. Preferably, said mixed alcohol waste comprises: C1-C4 saturated monohydric alcohols, water, and at least one of low-boiling esters with a boiling point of 50-90°C, acetic acid, and methyl glycolate, and at least one of polymers thereof.

Wherein, C1-C4 saturated monohydric alcohols can be, for example, methanol and ethanol; low-boiling esters with a boiling point of 50-90°C can be, for example, methyl acetate and ethyl acetate.

Furthermore, said mixed alcohol waste may also include one or more of esters, ethers, and ketones with a boiling point of 100-150°C. Said esters with a boiling point of 100-150°C do not include methyl glycolate.

According to one embodiment of the aforementioned first aspect, said mixed alcohol waste can be a by-product from a polyglycolic acid ester production process. In said mixed alcohol waste, the content of C1-C4 saturated monohydric alcohols is 30-70 wt%, preferably 40-69 wt%, for example 45 wt%, 50 wt%, 55 wt%, and 60 wt%; the content of low-boiling esters with a boiling point of 50-90°C is 1-9 wt%, preferably 1-7 wt%; the water content is 4-35 wt%, preferably 4-30 wt%; the acetic acid content is 1-15 wt%, preferably 1-10 wt%; the total content of esters, ethers, and ketones with a boiling point of 100-150°C is 1-30 wt%, preferably 1-20 wt%; and the methyl glycolate content is 1-41 wt%, preferably 10-40 wt%.

According to one embodiment of the aforementioned first aspect, high-temperature treatment that may be performed on the mixed alcohol waste before use might lead to the generation of some polymers. Therefore, said mixed alcohol waste can comprise polymers generated by polymerization of at least one of C1-C4 saturated monohydric alcohols, low-boiling esters with a boiling point of 50-90°C, acetic acid, methyl glycolate, and esters, ethers, and ketones with a boiling point of 100-150°C. Preferably, the polymer content in said mixed alcohol waste is 0-10 wt%, preferably containing no polymers.

According to one embodiment of the aforementioned first aspect, said heavy alcohol waste can be a by-product from a dimethyl oxalate hydrogenation unit or production process. Preferably, said heavy alcohol waste comprises at least one of ethylene glycol, glycolic acid, and methyl glycolate, and polymers generated by polymerization of at least one of ethylene glycol, glycolic acid, and methyl glycolate. The ethylene glycol therein can be separated and recovered together with the glycolate product.

According to one embodiment of the aforementioned first aspect, said heavy alcohol waste can further comprise one or more of monohydric alcohols such as methanol, ethanol, butanol; dihydric alcohols such as propylene glycol, butanediol; ethers such as dimethyl ether, ethylene glycol monomethyl ether; esters such as methyl acetate, ethyl acetate; and ketones such as 1,4-dioxane-2,5-dione.

According to one embodiment of the aforementioned first aspect, based on the total weight of said mixed alcohol waste and said heavy alcohol waste, the content of polymers coming from said heavy alcohol waste is greater than or equal to 5 wt%, preferably 10-80 wt%, more preferably 20-70 wt%, for example 30 wt%, 40 wt%, 50 wt%, or 60 wt%.

According to one embodiment of the aforementioned first aspect, in said heavy alcohol waste, the ethylene glycol content is 15-75 wt%, preferably 30-70 wt%, for example 40 wt% and 50 wt%; the methyl glycolate content is 0-30 wt%, for example 10-29 wt%; the glycolic acid content is 0-10 wt%, for example 5-10 wt%, based on the total weight of said heavy alcohol waste.

According to one embodiment of the aforementioned first aspect, methods used for said preliminary separation include but are not limited to: rectification, distillation-concentration, adsorption, and extraction; preferably rectification is employed; more preferably rectification is performed at a temperature not higher than 165°C.

According to one embodiment of the aforementioned first aspect, in said preliminary separation step or step S1, said preliminary separation method is performed by rectification, comprising:

a. subjecting said mixed alcohol waste to a first rectification at a column top pressure of 20-201 kPa, for example 20-101 kPa or 101-201 kPa, and a temperature of 40-120°C, controlling the number of column plates of the first rectification to be 5-38 and the reflux ratio to be 0.1-12, to obtain said first light component and non-distilled material;

b. subjecting the non-distilled material obtained from the first rectification to a second rectification at a column top pressure of 20-121 kPa, for example 20-101 kPa or 101-121 kPa, and a temperature of 60-162°C, controlling the number of column plates of the second rectification to be 5-38 and the reflux ratio to be 0.1-12, to obtain said first intermediate component and said first heavy component.

The above preliminary separation method can be implemented in one rectification column or in two rectification columns. If said preliminary separation method is implemented in one rectification column, the non-distilled material produced by the first rectification is not taken out, but the rectification is performed intermittently or continuously by adjusting the column top pressure and temperature of the rectification column. If said preliminary separation method is implemented in two rectification columns, the non-distilled material produced by the first rectification needs to be taken out and then transferred to the second rectification column to conduct the second rectification.

In the above preliminary separation method, the obtained first light component primarily comprises light alcohols such as C1-C4 alcohols, e.g., methanol. Preferably, in said first light component, the methanol content is 80-100 wt%; in a more preferred case, the methanol content in said first light component is 85-100 wt%. Since said first light component contains a relatively high methanol content, it can serve as a solvent in reaction step 2 (or step S2) and participate in the reaction. The first intermediate component primarily comprises impurities such as water, acid, esters, ketones, and ethers; these substances are distilled off and then disposed of as waste. The first heavy component obtained as the column bottom product primarily comprises glycolate compounds, which are fed into the reaction unit to participate in the reaction or fed into the separation unit to be combined with the reaction product obtained in the reaction unit and then subjected to separation together.

According to one embodiment of the aforementioned first aspect, in said reaction step 2 or step S2, said optionally added alcohol is selected from saturated monohydric alcohols; preferably C1-C4 saturated monohydric alcohols, such as methanol and ethanol, and the glycolates in the corresponding recovered material containing glycolates are the glycolate of the corresponding alcohol, e.g., methyl glycolate and ethyl glycolate. Said added alcohol can be fresh alcohol or a light component from the product separation step 3 (or step S3) of the method according to the present invention (hereinafter referred to as "second light component").

Preferably, said added fresh alcohol is methanol; therefore, the glycolate in the corresponding recovered material containing glycolates is primarily methyl glycolate.

To further increase the reaction rate, preferably, in reaction step 2 or step S2, said reaction is performed in the presence of a catalyst for catalyzing alcoholysis reactions. Preferably, said catalyst is selected from materials containing metal oxides, said metal oxides preferably selected from one or more of calcium oxide, zinc oxide, copper oxide, aluminum oxide, and rare earth oxides (e.g., cerium oxide), preferably materials containing zinc oxide. According to a preferred embodiment, based on the total weight of the catalyst, said catalyst comprises: 20-80 wt% zinc oxide, 5-45 wt% copper oxide, and 1-30 wt% aluminum oxide; preferably, said catalyst further comprises 0.1-10 wt% rare earth element oxide. Preferably, said rare earth element is selected from at least one of lanthanum, cerium, zirconium, and neodymium; preferably, based on the total weight of the catalyst, said catalyst comprises: 40-70 wt% zinc oxide, 10-40 wt% copper oxide, 5-25 wt% aluminum oxide, and 1-8 wt% rare earth element oxide, for example, a catalyst comprising the following components: 67 wt% calcium oxide, 20 wt% copper oxide, 10 wt% aluminum oxide, 3 wt% cerium oxide; or comprising the following components: 67 wt% zinc oxide, 20 wt% copper oxide, 10 wt% aluminum oxide, 3 wt% cerium oxide. More preferably, in reaction step 2 or step S2, based on the amount of the heavy alcohol waste being 100g, the amount of the catalyst is 1-100g, i.e., the weight ratio of the catalyst amount to the heavy alcohol waste amount is 1-100:100.

According to one embodiment of the aforementioned first aspect, in the aforementioned reaction step 2 or step S2, depending on whether the preliminary separation step or step S1 of the mixed alcohol waste is performed, and according to the difference between the viscosity of the reaction mixture and the desired viscosity, the reaction mixture can comprise heavy alcohol waste, mixed alcohol waste (if preliminary separation step or step S1 is not performed) and/or the first light component coming from said preliminary separation step or step S1, optionally added fresh alcohol, and optionally a light component coming from the reaction product separation step or step S3; wherein, said optionally added alcohol primarily serves as a solvent to reduce the viscosity of said reaction mixture containing mixed alcohol waste and heavy alcohol waste. That is, if the viscosity of the reaction mixture is too high, additional alcohol solvent needs to be added; if the viscosity of the reaction mixture is suitable, no additional alcohol solvent can be needed. The additionally added alcohol can come from fresh alcohol or from a light component primarily comprising light alcohols from the reaction product separation step 3 or S3 (hereinafter also referred to as second light component). Therefore, according to a preferred embodiment, in the aforementioned reaction step 2 or step S2, said reaction mixture comprises heavy alcohol waste, the first light component from step S1, added fresh alcohol, and optionally a light component from the reaction product separation step 3 or S3; or, in the aforementioned reaction step 2 or step S2, the reaction mixture comprises heavy alcohol waste, the first light component and first heavy component from step S1, added alcohol, and optionally a light component from the reaction product separation step 3 or step S3.

When mixing heavy alcohol waste, added fresh alcohol, the first light component obtained in preliminary separation step 1 (or step S1), and optionally a light component from the reaction product separation step 3 or step S3 and allowing them to react, the amounts of said heavy alcohol waste, added alcohol, first light component, and light component from the reaction product separation step 3 or S3 are such that the weight ratio of the heavy alcohol waste to the total weight of the first light component and said added alcohol in the obtained mixture is 1:(1-7), preferably 1:(2-5).

According to one embodiment of the aforementioned first aspect, in reaction step 2 or step S2, when mixing heavy alcohol waste, added alcohol, the first light component obtained in preliminary separation step 1 (or step S1), and the first heavy component and allowing them to react, the amounts of said heavy alcohol waste, added alcohol, first light component, and first heavy component are such that the weight ratio of the heavy alcohol waste : the total weight of the added alcohol and first light component : the weight of the first heavy component is 1:(1-7):(0.01-0.5), preferably 1:(2-5):(0.1-0.4).

According to one embodiment of the aforementioned first aspect, preferably, in said reaction step or step S2, the pressure of the reaction is 0.1-6 MPa; the temperature of the reaction is not higher than the boiling points of the components in the mixed alcohol waste and heavy alcohol waste and their azeotropes under the same pressure conditions, preferably 130-230°C, more preferably 160-220°C; the reaction time is 10 minutes to 4 hours, preferably 0.5 hours to 3 hours.

According to one embodiment of the aforementioned first aspect, in said product separation step 3 or step S3, the method for separating the material containing glycolates includes but is not limited to: reduced pressure rectification, atmospheric pressure rectification, atmospheric pressure distillation, reduced pressure distillation, and pressure distillation; preferably reduced pressure rectification or atmospheric pressure rectification; more preferably reduced pressure rectification or atmospheric pressure rectification at a temperature not higher than 160°C.

According to one embodiment of the aforementioned first aspect, in said separation product step or step S3, said separation method comprises the following steps:

A. subjecting the reaction product obtained in said reaction step or step S2 to a third rectification at a column top pressure of 20-201 kPa, for example 20-101 kPa or 101-201 kPa, and a column top temperature of 50-160°C, preferably 70-140°C, controlling the number of column plates of the third rectification to be 5-42 and the reflux ratio to

be 1-15, to separate out a second light component;

B. subjecting the non-distilled material obtained from the third rectification to a fourth rectification under conditions of a column top pressure of 5 Pa - 101 kPa and a column top temperature of 70-170°C, controlling the number of column plates of the fourth rectification to be 5-42 and the reflux ratio to be 1-15, to separate out a material containing glycolates, referred to as "second intermediate component"; the material remaining in the column bottom primarily comprises undecomposed polymers, referred to as "second heavy component".

The above reaction product separation method can be implemented in one rectification column or in two rectification columns. If said preliminary separation method is implemented in one rectification column, the non-distilled material produced by the first rectification is not taken out, but the rectification is performed intermittently or continuously by adjusting the column top pressure and temperature of the rectification column. If said reaction product separation method is implemented in two rectification columns, the non-distilled material from the third rectification needs to be taken out and then transferred to the second rectification column to perform the fourth rectification.

Typically, parameters such as column top temperature, column bottom temperature, and column top pressure of the rectification column are adjusted and selected by technicians within the above ranges based on actual conditions; however, according to one embodiment, the difference $\Delta T$ between the column top temperatures of the second rectification and the first rectification can be greater than 15°C, e.g., 20-50°C; the difference $\Delta T$ between the column top temperatures of the fourth rectification and the third rectification can be greater than 30°C, e.g., 40-90°C; and/or, the difference $\Delta T$ between the column bottom temperatures of the second rectification and the first rectification can be greater than 15°C, e.g., 20-50°C; the difference $\Delta T$ between the column bottom temperatures of the fourth rectification and the third rectification can be greater than 30°C, e.g., 40-90°C; and/or, the difference $\Delta P$ between the column top pressures of the first rectification and the second rectification can be greater than 5 kPa, e.g., 10-80 kPa; the difference $\Delta P$ between the column top pressures of the third rectification and the fourth rectification can be greater than 10 kPa, e.g., 15-90 kPa.

In the above preliminary separation method, the second light component primarily comprises light alcohols such as C1-C4 alcohols, e.g., methanol. Since said second light component contains a relatively high alcohol content, it can serve as a solvent and be returned to reaction step 2 or step S2 together with added fresh alcohol to participate in the reaction.

According to one embodiment of the aforementioned first aspect, said joint treatment method further comprises: returning at least a portion of the residual material after separating out said material containing glycolates to reaction step 2 or step S2 to perform reaction; and, returning at least a portion of the second light component distilled after the third rectification as one source of alcohol to said reaction step 2 or step S2 to participate in the reaction, and subjecting at least a portion of the not distilled heavy component (referred to as "second heavy component") primarily comprising residual polymers after the fourth rectification to further conversion as waste to be treated.

Referring to FIG. 1 to illustrate a preferred embodiment of the joint treatment method for a mixed alcohol waste and a heavy alcohol waste according to the present invention, wherein said joint treatment method comprises the following steps: introducing mixed alcohol waste into a preliminary separation unit to perform preliminary purification separation, respectively obtaining a first light component primarily comprising light alcohols, a first intermediate component containing water, acid, and low-boiling ester, ketone, and ether impurities, and a first heavy component comprising high-boiling esters (primarily being glycolates and possibly polymers thereof); wherein, the first light component is fed as a solvent to the reaction unit, the first intermediate component is discarded as waste, and the first heavy component is fed into the product separation unit; feeding heavy alcohol waste and added alcohol (including fresh methanol and a second light component from the product separation unit) to the reaction unit, where they are mixed with the first light component and allowed to react; the obtained reaction product is combined with said first heavy component and then fed into the product separation unit to perform separation, respectively obtaining a second light component primarily comprising lower alcohols, a second intermediate component (material primarily containing glycolates and/or ethylene glycol), and a second heavy component (primarily comprising unreacted polymers); the second intermediate component is recovered as product; and at least a portion of the second light component and at least a portion of the second heavy component are returned to the reaction unit to participate in the reaction.

Referring to FIG. 2 to illustrate another preferred embodiment of the joint treatment method for a mixed alcohol waste and a heavy alcohol waste according to the present invention, wherein said joint treatment method comprises the following steps: introducing mixed alcohol waste into a preliminary separation unit to perform preliminary separation, respectively obtaining a first light component primarily comprising light alcohols, a first intermediate component containing water, acid, and low-boiling ester, ketone, and ether impurities, and a first heavy component comprising high-boiling esters (primarily being glycolates and possibly polymers thereof); wherein, said first light component and first heavy component are fed into the reaction unit, and the first intermediate component is discarded as waste. Heavy alcohol waste, added alcohol (including fresh methanol and a second light component from the product separation unit), the first light component, and the first heavy component are mixed in the reaction unit and allowed to react; the obtained reaction

product is fed into the product separation unit to perform separation, respectively obtaining a second light component comprising lower alcohols, a second intermediate component (material containing glycolates and/or ethylene glycol), and a second heavy component (primarily comprising unreacted polymers); the second intermediate component is recovered as product; and at least a portion of the second light component and at least a portion of the second heavy component are returned to the reaction unit to participate in the reaction.

According to the second aspect of the present invention, the present invention also provides a system for implementing the joint treatment method according to the first aspect of the present invention, comprising: an optional preliminary separation unit, a reaction unit, and a product separation unit, wherein said preliminary separation unit is used to remove water and acid in the mixed alcohol waste and optionally separate out a first heavy component primarily comprising high-boiling esters, to obtain a preliminary purification product; said reaction unit is used to mix heavy alcohol waste, said preliminary purification product, and optionally added alcohol and allow them to react; and said product separation unit is used to separate a material containing glycolates and/or ethylene glycol from the reaction product.

According to one embodiment of the aforementioned second aspect of the present invention, said preliminary separation unit comprises one or more rectification columns; said reaction unit comprises one or more reaction kettles; said product separation unit comprises one or more rectification columns.

According to one embodiment of the aforementioned second aspect, said preliminary separation unit comprises two rectification columns connected in series, namely a first rectification column and a second rectification column; said reaction unit comprises a reaction kettle; said product separation unit comprises two rectification columns connected in series, namely a third rectification column and a fourth rectification column.

Examples

The technical solutions of the present invention and the obtained technical effects will be illustrated by the following examples and comparative examples. In the following examples and comparative examples, unless otherwise specified, the involved operation methods and parameter measurement methods are conventional methods in the field, and the reagents, materials, or raw materials used are all available commercially.

The following two catalysts were used in the examples:

Catalyst 1: Zinc oxide 40 wt%, copper oxide 40 wt%, aluminum oxide 19 wt%, cerium oxide 1 wt%;

Catalyst 2: Zinc oxide 70 wt%, copper oxide 10 wt%, aluminum oxide 15 wt%, lanthanum oxide 5 wt%.

The composition of materials or products was determined and calculated by gas chromatography according to the normalization method. The types and contents of various components were determined by chromatographic peaks. Wherein although some ester, ketone, and ether impurities, polymers, and some high-boiling components in the heavy component were difficult to identify their specific molecular structures, their retention times in the spectrum were fixed. The content of polymers changed significantly after the reaction, while the contents of other substances difficult to identify their molecular structures did not change before and after the reaction, thus determining the types and content of each component. In the following examples, various reaction parameters were calculated as follows:

Product (i.e., glycolate and ethylene glycol) yield (%) = Total weight of glycolate and ethylene glycol in the recovered product containing glycolate and ethylene glycol ÷ (Total weight of methyl glycolate and ethylene glycol in the heavy alcohol waste + weight of methyl glycolate in the mixed alcohol waste) × 100%;

Polymer conversion rate (%) = (Weight of polymers in the heavy alcohol waste and mixed alcohol waste - Weight of polymers in the reaction product) ÷ Weight of polymers in the heavy alcohol waste and mixed alcohol waste × 100%;

Glycolate selectivity (%) = (Weight of glycolates in the reaction product - Weight of glycolates in the heavy alcohol waste and mixed alcohol waste) ÷ (Weight of polymers in the heavy alcohol waste and mixed alcohol waste - Weight of polymers in the reaction product) × 100%;

Ethylene glycol selectivity (%) = (Weight of ethylene glycol in the reaction product - Weight of ethylene glycol in the heavy alcohol waste and mixed alcohol waste) ÷ (Weight of polymers in the heavy alcohol waste and mixed alcohol waste - Weight of polymers in the reaction product) × 100%.

Example 1

A mixed alcohol waste with a mass flow rate of 975 kg/h was fed into a first rectification column of a preliminary separation unit, said mixed alcohol waste comprising: ethanol 1.45 wt%, methanol 36 wt%, water 10 wt%, methyl glycolate 40.76 wt%, acetic acid 3.12 wt%, methyl acetate 2.15 wt%, ester, ether, ketone impurities 6.52 wt%. The column top temperature of the first rectification column was 71.8°C, the column bottom temperature was 110°C, the column top pressure was 35 kPa, the number of column plates was 20, and the reflux ratio at column top was 5. A first light component with a mass flow rate of 375 kg/h was taken from the column top, comprising 91.2 wt% methanol, 3.3 wt% ethanol, and 5.5 wt% methyl acetate. This first light component was fed into the reaction unit as a solvent. The column bottom material from the first rectification column was fed into a second rectification column of the preliminary separation unit. The column top temperature of the second rectification column was 98.2°C, the column bottom temperature was 159.8°C, the column top pressure was 25 kPa, the number of column plates was 20, and the reflux ratio at column top was 3. A first intermediate component with a mass flow rate of 200 kg/h was taken from the column top, containing water, acid, ester, ketone, ether impurities. A first heavy component rich in methyl glycolate with a mass flow rate of 400 kg/h was taken from the column bottom of the second rectification column, which was then fed into the product separation unit.

A mass flow rate of 5650 kg/h of heavy alcohol waste with, 2665 kg/h of methanol, and a total mass flow rate of 26950 kg/h of the first light component from the preliminary separation unit and a second light component from the product separation unit were fed into a reaction kettle of the reaction unit, the reaction kettle containing a catalyst with the following composition: zinc oxide 40 wt%, copper oxide 40 wt%, aluminum oxide 19 wt%, cerium oxide 1 wt%. Wherein the heavy alcohol waste comprised: methyl glycolate 2.5 wt%, ethylene glycol 20.54 wt%, polymer-rich component 76.96 wt%. The temperature of the reaction kettle was 180°C, the pressure was 2 MPa, and the residence time of the reaction material was 1 hour, obtaining a liquid phase product with a mass flow rate of 35650 kg/h, the product comprising: methanol 76.26 wt%, methyl glycolate 10.03 wt%, ethylene glycol 11.18 wt%, heavy components (including unreacted polymers and substances whose molecular structures were not identified but with boiling points greater than or equal to 200°C and remaining in the column bottom of rectification columns of the product separation unit) 2.53 wt%.

Said liquid phase product was combined with the first heavy component from the second rectification column and fed into a third rectification column of the product separation unit. The column top temperature of the third rectification column was 72.1°C, the column bottom temperature was 138.1°C, the pressure was 35 kPa, the number of column plates was 20, and the reflux ratio at column top was 2. A second light component with a mass flow rate of 27000 kg/h was taken from the column top, which was methanol with a mass purity of 98.7 wt%; wherein 26950 kg/h of the second light component was returned to said reaction unit to act as a circulating solvent, and 50 kg/h was taken out from the system. A liquid phase material with a mass flow rate of 9050 kg/h was taken from the column bottom of the third rectification column and fed into a fourth rectification column of said product separation unit. The column top temperature of the fourth rectification column was 121.2°C, the column bottom temperature was 159°C, the pressure was 10 kPa, the number of column plates was 20, and the reflux ratio at column top was 3. A second intermediate component with a mass flow rate of 8600 kg/h was taken from the column top of the fourth rectification column, the second intermediate component comprising 46.2 wt% methyl glycolate and 45.5 wt% ethylene glycol. The second intermediate component was recovered as product, wherein the combined mass purity of these two compounds being 91.7 wt%. By calculation, the product yield of this example was approximately 464%, the polymer conversion rate (%) was 93.5%; the glycolate selectivity (%) was 106.9%; and the ethylene glycol selectivity (%) was 88.9%. Simultaneously, a heavy component of 450 kg/h was obtained from the column bottom of the fourth rectification column.

Example 2

A mixed alcohol waste with a mass flow rate of 975 kg/h was fed into a first rectification column of a preliminary separation unit, wherein said mixed alcohol waste comprised: ethanol 3.2 wt%, methanol 37 wt%, water 19 wt%, methyl glycolate 27.13 wt%, acetic acid 3.12 wt%, methyl acetate 2.2 wt%, ester, ketone, ether impurities 8.35 wt%. The column top temperature of the first rectification column was 72°C, the column bottom temperature was 95.2°C, the column top pressure was 35 kPa, the number of column plates was 20, and the reflux ratio at column top was 5. A first light component with a mass flow rate of 380 kg/h was taken from the column top, comprising 92.5 wt% methanol, 1.6 wt% ethanol, and 5.9 wt% methyl acetate. This first light component was fed into the reaction unit as a solvent. The column bottom material from the first rectification column was fed into a second rectification column of the preliminary separation unit. The column top temperature of the second rectification column was 104°C, the column bottom temperature was 161.6°C, the column top pressure was 25 kPa, the number of column plates was 20, and the reflux ratio at column top was 3. A first intermediate component with a mass flow rate of 270 kg/h was taken from the column top, containing water, acid, ester, ketone, ether impurities. A first heavy component rich in methyl glycolate with a mass flow rate of 325 kg/h was taken from the column bottom of the second rectification column, which was then fed into the product separation unit.

Heavy alcohol waste with a mass flow rate of 5650 kg/h and having the same composition as the heavy alcohol

waste in Example 1, 2680 kg/h methanol, and a total mass flow rate of 26650 kg/h of the first light component from the preliminary separation unit and a second light component from the product separation unit were fed into a reaction kettle containing catalyst of the reaction unit. Said catalyst had the following composition: zinc oxide 70 wt%, copper oxide 10 wt%, aluminum oxide 15 wt%, lanthanum oxide 5 wt%. The temperature of the reaction kettle was 200°C, the pressure was 2 MPa, and the residence time of the reaction material was 2 hours, obtaining a liquid phase product with a mass flow rate of 35350 kg/h, comprising: methanol 75.3 wt%, methyl glycolate 10.53 wt%, ethylene glycol 11.68 wt%, heavy components (including unreacted polymers and substances whose molecular structures were not identified but with boiling points greater than or equal to 200°C and remaining in the rectification column bottom of the product separation unit) 2.49 wt%.

Said liquid phase product was combined with the first heavy component from the second rectification column and fed into said third rectification column of the product separation unit. The column top temperature of the third rectification column was 72.1°C, the column bottom temperature was 147.1°C, the pressure was 35 kPa, the number of column plates was 20, and the reflux ratio at column top was 2. A second light component with a mass flow rate of 26700 kg/h was taken from the column top, which was methanol with a mass purity of 98.7 wt%, wherein 26650 kg/h of the second light component was returned to said reaction unit to act as a circulating solvent, and 50 kg/h of the second light component was taken out from the system. A liquid phase material with a mass flow rate of 8975 kg/h was taken from the column bottom of the third rectification column and fed into said fourth rectification column of the product separation unit. The column top temperature of the fourth rectification column was 121.2°C, the column bottom temperature was 158°C, the column top pressure was 10 kPa, the number of column plates was 20, and the reflux ratio at column top was 3. A second intermediate component with a mass flow rate of 8550 kg/h was taken from the column top of the fourth rectification column, comprising 46.4 wt% methyl glycolate and 48.1 wt% ethylene glycol. The second intermediate component was recovered as product, with a combined mass purity of these two compounds being 94.5 wt%. By calculation, the glycolate selectivity (%) was 110.3%; the ethylene glycol selectivity (%) was 92.3%; the product yield of this example was approximately 515.8%. Simultaneously, a heavy component of 425 kg/h was obtained from the column bottom of the fourth rectification column.

Example 3

A mixed alcohol waste with a mass flow rate of 975 kg/h was fed into a first rectification column of a preliminary separation unit, wherein said mixed alcohol waste comprised: ethanol 1 wt%, methanol 42 wt%, water 10 wt%, methyl glycolate 34.76 wt%, acetic acid 3.12 wt%, methyl acetate 2.6 wt%, ester, ketone, ether impurities 6.52 wt%. The column top temperature of the first rectification column was 71°C, the column bottom temperature was 104°C, the column top pressure was 35 kPa, the number of column plates was 20, and the reflux ratio at column top was 5. A first light component with a mass flow rate of 435 kg/h was taken from the column top, comprising 93.1 wt% methanol, 1.0 wt% ethanol, and 5.9 wt% methyl acetate. This first light component was fed into the reaction unit as a solvent. The column bottom material from the first rectification column was fed into a second rectification column of the preliminary separation unit. The column top temperature of the second rectification column was 102°C, the column bottom temperature was 159°C, the column top pressure was 25 kPa, the number of column plates was 20, and the reflux ratio at column top was 3. A first intermediate component with a mass flow rate of 200 kg/h was taken from the column top, containing water, acid, ester, ketone, ether impurities. A first heavy component rich in methyl glycolate with a mass flow rate of 340 kg/h was taken from the column bottom of the second rectification column, which was then fed into the reaction unit.

A heavy alcohol waste with a mass flow rate of 5650 kg/h, 3200 kg/h methanol, 340 kg/h of the first heavy component rich in methyl glycolate, and a total mass flow rate of 26450 kg/h of the first light component from the preliminary separation unit and a second light component from the product separation unit were fed into a reaction kettle of the reaction unit, wherein the heavy alcohol waste comprised: methyl glycolate 10.2 wt%, ethylene glycol 14 wt%, polymer-rich component 75.8 wt%. The reaction kettle contained 2 kg of catalyst having the following composition: zinc oxide 40 wt%, copper oxide 40 wt%, aluminum oxide 19 wt%, cerium oxide 1 wt%. The temperature of the reaction kettle was 190°C, the pressure was 2.2 MPa, and the residence time of the reaction material was 1 hour, obtaining a liquid phase product with a mass flow rate of 35735 kg/h, comprising: methanol 72 wt%, methyl glycolate 14.29 wt%, ethylene glycol 11.18 wt%, heavy components (including unreacted polymers and substances whose molecular structures were not identified but with boiling points greater than or equal to 200°C and remaining in the rectification column bottom of the product separation unit) 2.53 wt%.

Said liquid phase product was fed into the third rectification column of the product separation unit. The column top temperature of the third rectification column was 71°C, the column bottom temperature was 153°C, the column top pressure was 80 kPa, the number of column plates was 20, and the reflux ratio at column top was 2. A second light component with a mass flow rate of 26500 kg/h was taken from the column top, which was methanol with a purity of 97 wt%, wherein 26450 kg/h of the second light component was returned to said reaction unit to act as a circulating solvent, and 50 kg/h of the second light component was taken out from the system. A liquid phase material with a mass flow rate of 9575 kg/h was taken from the column bottom of the third rectification column and fed into the fourth rectification column of said product separation unit. The column top temperature of the fourth rectification column was 123°C, the column bottom

temperature was 149°C, the pressure was 10 kPa, the number of column plates was 20, and the reflux ratio at column top was 3. A second intermediate component with a mass flow rate of 9188 kg/h was taken from the column top of the fourth rectification column, comprising 55.4 wt% methyl glycolate and 43.1 wt% ethylene glycol; the second intermediate component was recovered as product, with a combined purity of these two compounds being 98.5 wt%. By calculation, the product yield of this example was approximately 531.4%, and the polymer conversion rate (%) was 95.9%. The selectivity for methyl glycolate and ethylene glycol in the obtained product was approximately 116.0% and 88.7%, respectively. Simultaneously, a heavy component of 387 kg/h was obtained from the column bottom of the fourth rectification column.

Example 4

This example was operated according to the method of Example 1, except that the reaction step was carried out in a reaction kettle without a catalyst. Based on measurement and analysis of the final product, the product yield of this example was approximately 274%, the polymer conversion rate was approximately 67.6%, and the selectivity for methyl glycolate and ethylene glycol in the obtained product was approximately 78.4% and 38.3%, respectively.

Comparative Example 1

This comparative example was conducted according to the procedure of Example 3, except that the mixed alcohol waste and heavy alcohol waste were reacted separately, and the total product yield was calculated.

A mixed alcohol waste with a mass flow rate of 975 kg/h was fed into a first rectification column of a preliminary separation unit, wherein said mixed alcohol waste comprised: ethanol 1 wt%, methanol 42 wt%, water 10 wt%, methyl glycolate 34.76 wt%, acetic acid 3.12 wt%, methyl acetate 2.6 wt%, ester, ketone, ether impurities 6.52 wt%. The column top temperature of the first rectification column was 71°C, the column bottom temperature was 104°C, the pressure was 35 kPa, the number of column plates was 20, and the reflux ratio at column top was 5. A first light component with a mass flow rate of 435 kg/h was taken from the column top, comprising 93.1 wt% methanol, 1.0 wt% ethanol, and 5.9 wt% methyl acetate. This first light component was fed into the reaction unit as a solvent. The column bottom material from the first rectification column was fed into a second rectification column of the preliminary separation unit. The column top temperature of the second rectification column was 102°C, the column bottom temperature was 159°C, the pressure was 25 kPa, the number of column plates was 20, and the reflux ratio at column top was 3. A first intermediate component with a mass flow rate of 200 kg/h was taken from the column top, containing water, acid, ester, ketone, ether impurities. A first heavy component rich in methyl glycolate with a mass flow rate of 340 kg/h was taken from the column bottom of the second rectification column.

Then, the aforementioned first light component and the first heavy component rich in methyl glycolate were mixed and fed into a reaction kettle containing catalyst, the composition of the catalyst being: zinc oxide 40 wt%, copper oxide 40 wt%, aluminum oxide 19 wt%, cerium oxide 1 wt%. A first liquid phase product of 775 kg/h was obtained, comprising approximately 33.77 wt% methyl glycolate.

A heavy alcohol waste with a mass flow rate of 5650 kg/h and having the same composition as in Example 1, 3500 kg/h methanol, and a total mass flow rate of 27950 kg/h of a second light component from the product separation unit were fed into a reaction kettle containing catalyst in the reaction unit, said catalyst having the following composition: zinc oxide 40 wt%, copper oxide 40 wt%, aluminum oxide 19 wt%, cerium oxide 1 wt%. The heavy alcohol waste comprised: methyl glycolate 2.5 wt%, ethylene glycol 20.54 wt%, polymer-rich component 76.96 wt%. The temperature of the reaction kettle was 180°C, the pressure was 2 MPa, and the residence time of the reaction material was 1 hour, obtaining a second liquid phase product with a mass flow rate of 37100 kg/h, comprising approximately 9.70 wt% methyl glycolate, approximately 10.24 wt% ethylene glycol, with the remainder primarily being methanol.

Said first liquid phase product and second liquid phase product were mixed and fed into a third rectification column of the product separation unit. The column top temperature of the third rectification column was 72.1°C, the column bottom temperature was 140.2°C, the pressure was 35 kPa, the number of column plates was 20, and the reflux ratio at column top was 2. A second light component with a mass flow rate of 28000 kg/h was taken from the column top, which was methanol with a mass purity of 99.4 wt%, of which 27950 kg/h was returned to said reaction unit to act as a circulating solvent, and of which 200 kg/h was taken out from the system. A liquid phase material with a mass flow rate of 9875 kg/h was taken from the column bottom of the third rectification column and fed into a fourth rectification column of said product separation unit. The column top temperature of the fourth rectification column was 121.2°C, the column bottom temperature was 159°C, the pressure was 10 kPa, the number of column plates was 20, and the reflux ratio at column top was 3. A second intermediate component with a mass flow rate of 8700 kg/h was taken from the column top of the fourth rectification column, comprising 44.2 wt% methyl glycolate and 39.3 wt% ethylene glycol; the second intermediate component was recovered as product, with a combined mass purity of these two compounds being 83.5 wt%. By calculation, the product yield of this example was approximately 443%, and the polymer conversion rate (%) was approximately 90%. The selectivity for methyl glycolate and ethylene glycol in the obtained product was approximately

100.9% and 85.3%, respectively.

The schemes and results of the above example are listed in Table 1.

Table 1.

| | scheme | catalyst | Polymer conversion rate (%) | Product yield (%) | methyl glycolate selectivity (%) | ethylene glycol selectivity (%) |
|---|---|---|---|---|---|---|
| Ex. 1 | Joint processing scheme 1 | Catalyst 1 | 93.5 | 464 | 106.9 | 88.9 |
| Ex. 2 | Joint processing scheme 1 | Catalyst 2 | 94.5 | 515.8 | 110.3 | 92.3 |
| Ex. 3 | Joint processing scheme 2 | Catalyst 1 | 95.9 | 531.4 | 116.0 | 88.7 |
| Ex. 4 | Joint processing scheme 1 | Catalyst-free | 67.6 | 274 | 78.4 | 38.3 |
| Comp. Ex. 1 | Mixed alcohol waste and heavy alcohol waste are treated separately. | Catalyst 1 | 90.0 | 443 | 100.9 | 85.3 |

From the results of the above examples, it can be seen that Examples 1 and 2 of the present invention used the method shown in FIG. 1 (Joint Treatment Scheme 1) to treat mixed alcohol waste and heavy alcohol waste. The first heavy component obtained from preliminary separation of the mixed alcohol waste did not participate in the reaction but was combined with the reaction product from the reaction step and then was separated. Example 3 used the method shown in FIG. 2 (Joint Treatment Scheme 2) to treat mixed alcohol waste and heavy alcohol waste. The first heavy component obtained from preliminary separation of the mixed alcohol waste participated in the reaction in the reaction step, and then the reaction product was separated. Both the two treatment methods could achieve very high product yields while achieving efficient recovery of mixed alcohol waste and heavy alcohol waste. Additionally, compared to Example 1, Example 2 used a different catalyst and increased the reaction temperature, achieving higher polymer conversion rates and better product yields. Furthermore, Example 3 using Joint Treatment Scheme 2, compared to Examples 1 and 2 using Joint Treatment Scheme 1, obtained a higher product yield, possibly because the column bottom material (the stream rich in methyl glycolate) left after the two rectifications of the mixed alcohol waste contained a portion of glycolate polymers. By entering the reaction unit to perform reaction, the portion of glycolate polymers could be converted, thereby achieving a higher product yield. In the examples of the present invention, the product yield (including ethylene glycol and glycolates) was far higher than 100%, this is because the polymers (primarily glycolate polymers) and other components in the mixed alcohol waste and heavy alcohol waste produced ethylene glycol and glycolates through alcoholysis and depolymerization reactions, causing the content of ethylene glycol and glycolates in the product stream to be far higher than the content contained in the initial mixed alcohol waste and heavy alcohol waste.

By comparing Examples 1-3 with Example 4, it can be seen that in the reaction step of the method of the present invention, if no catalyst was used, conversion and alcoholysis reactions of polymers in the mixture of mixed alcohol waste and heavy alcohol waste can be achieved, but the polymer conversion rate (%) and product yield (%) are significantly lower, and the methyl glycolate selectivity (%) and ethylene glycol selectivity (%) are also significantly lower. Therefore, the use of the catalyst according to the present invention is preferred in the reaction process of the mixture of mixed alcohol waste and heavy alcohol waste.

By comparing Example 3 and Comparative Example 1, it can be seen that when mixed alcohol waste and heavy alcohol waste are treated jointly, compared to treating them separately, the polymers contained in these two wastes have significantly higher conversion rates, producing more methyl glycolate and ethylene glycol, thus significantly increasing the product yield. Moreover, separate treatment of mixed alcohol waste and heavy alcohol waste requires the use of more fresh alcohol than the joint treatment of them to dissolve the heavy alcohol waste and carry out the alcoholysis reaction. Additionally, the two products produced by the reaction of mixed alcohol waste and the reaction of heavy alcohol waste both require distillation separation, consuming more energy than the joint treatment method, involving more separation steps and generating higher production costs.

The preferred embodiments of the present invention have been described in detail above, but the present invention is not limited thereto. Within the technical concept scope of the present invention, various simple modifications can be made to the technical solutions of the present invention, including combining various technical features in any other

suitable manner. These simple modifications and combinations should also be considered as disclosed content of the present invention and fall within the protection scope of the present invention.

## Claims

1. A joint treatment method for a mixed alcohol waste and a heavy alcohol waste, **characterized in that** said joint treatment method comprises the following steps:

   Step 1. optionally, subjecting the mixed alcohol waste primarily comprising C1-C4 saturated monohydric alcohols to preliminary separation treatment to at least partially remove contained water and acid, and optionally at least partially separating out a heavy component primarily comprising esters, to obtain a preliminary purification product of said mixed alcohol waste;
   Step 2. mixing the mixed alcohol waste that optionally has been preliminarily separated, said heavy alcohol waste, and optionally added alcohol, and allowing them to react in the presence of an optional catalyst, to obtain a product containing glycolates and ethylene glycol; and optionally
   Step 3. a step of separating a material containing glycolates and ethylene glycol from the product obtained in Step 2 and optionally from the heavy component obtained in Step 1;
   wherein said heavy alcohol waste comprises at least one of ethylene glycol, glycolic acid, and methyl glycolate, and polymers produced by polymerization reaction of at least one of these compounds;

2. The joint treatment method according to claim 1, **characterized in that** said joint treatment method comprises the following steps:

   S1. optionally, subjecting said mixed alcohol waste to preliminary separation to at least partially separate out a heavy component primarily comprising esters, referred to as "first heavy component", and at least partially remove water and acid contained in said mixed alcohol waste, referred to as "first intermediate component", to obtain a preliminary purification product of said mixed alcohol waste, referred to as "first light component";
   S2. mixing said heavy alcohol waste, optionally added alcohol, and either said mixed alcohol waste not subjected to preliminary separation treatment or said preliminary purification product of the mixed alcohol waste obtained in step S1, in the presence of an optional catalyst, to conduct reaction; and
   S3. combining the reaction product obtained in step S2 and the first heavy component obtained in step S1, and separating therefrom a material containing glycolates and ethylene glycol.

3. The joint treatment method according to claim 1, **characterized in that** said joint treatment method comprises the following steps:

   S1. optionally, subjecting said mixed alcohol waste to preliminary separation treatment to at least partially separate out a heavy component primarily comprising esters, referred to as "first heavy component", and at least partially remove water and acid contained in said mixed alcohol waste, referred to as "first intermediate component", to obtain a preliminary purification product of said mixed alcohol waste, referred to as "first light component";
   S2. mixing said heavy alcohol waste, optionally added alcohol, and either said mixed alcohol waste not subjected to preliminary separation treatment or said preliminary purification product of the mixed alcohol waste and said first heavy component obtained in step S1, in the presence of an optional catalyst, to perform reaction; and
   S3. separating a material containing glycolates and ethylene glycol from the reaction product obtained in step S2.

4. The joint treatment method according to any one of claims 1-3, **characterized in that** said mixed alcohol waste comprises: C1-C4 saturated monohydric alcohols, water, and at least one of low-boiling esters with a boiling point of 50-90°C, acetic acid, and methyl glycolate, and optionally at least one of esters, ethers, and ketones with a boiling point of 100-150°C; preferably, in said mixed alcohol waste, the content of C1-C4 saturated monohydric alcohols is 30-70 wt%, the content of low-boiling esters with a boiling point of 50-90°C is 1-9 wt%, the water content is 5-35 wt%, the acetic acid content is 1-15 wt%, the total content of esters, ethers, and ketones with a boiling point of 100-150°C is 1-30 wt%, and the methyl glycolate content is 1-41 wt%.

5. The joint treatment method according to any one of claims 1-4, **characterized in that**, based on the total weight of said mixed alcohol waste and said heavy alcohol waste, the content of polymers from said heavy alcohol waste is greater than or equal to 5 wt%, preferably 10-80 wt%; and/or, based on the weight of said heavy alcohol waste, the ethylene

glycol content in said heavy alcohol waste is 15-75 wt%, the methyl glycolate content is 0-30 wt%, and the glycolic acid content is 0-10 wt%.

**6.** The joint treatment method according to any one of claims 1-5, **characterized in that** said Step 1 or step S1 comprises:

a. subjecting said mixed alcohol waste to a first rectification at a column top pressure of 101-201 kPa and a column top temperature of 40-120°C; or, subjecting said mixed alcohol waste to a first rectification at a column top pressure of 20-101 kPa and a column top temperature of 40-110°C, controlling the number of column plates of the first rectification to be 5-38 and the reflux ratio to be 0.1-12, to obtain said first light component and non-distilled material;
b. subjecting the non-distilled material obtained from the first rectification to a second rectification at a column top pressure of 101-121 kPa and a column top temperature of 60-160°C; or, subjecting the non-distilled material obtained from the first rectification to a second rectification at a column top pressure of 20-101 kPa and a column top temperature of 50-162°C, controlling the number of column plates of the second rectification to be 5-38 and the reflux ratio to be 0.1-12, to obtain said first intermediate component and said first heavy component.

**7.** The joint treatment method according to any one of claims 1-6, **characterized in that**, in the reaction step, said added alcohol is selected from saturated monohydric alcohols, preferably C1-C4 saturated monohydric alcohols, more preferably methanol.

**8.** The joint treatment method according to any one of claims 1-7, **characterized in that**, in the reaction step, said reaction is performed in the presence of a catalyst; said catalyst is selected from materials containing zinc oxide and/or materials containing copper oxide; preferably, said catalyst comprises 20-80 wt% zinc oxide, 5-45 wt% copper oxide, and 1-30 wt% aluminum oxide; preferably, the weight ratio of the amount of catalyst to the amount of heavy alcohol waste is 1-100:100.

**9.** The joint treatment method according to any one of claims 2 and 4-8, **characterized in that**, in the S2 reaction step, the amounts of said heavy alcohol waste, added alcohol, and the preliminary purification product obtained in S1 are such that the ratio of the weight of the heavy alcohol waste to the total weight of all alcohols is 1:(1-7); and/or, in said S2 reaction step, the reaction conditions include: a pressure of 0.1-6 MPa, a temperature of 160-220°C, and a time of 10 minutes to 4 hours.

**10.** The joint treatment method according to any one of claims 3-8, **characterized in that**, in the reaction step S2, the amounts of heavy alcohol waste, added alcohol, the preliminary purification product obtained in S1, and the first heavy component are such that the ratio of the weight of the first heavy alcohol waste : the total weight of all alcohols : the weight of heavy component is 1:(1-7):(0.01-0.5); and/or, in said S2 reaction step, the reaction conditions include: a pressure of 0.1-6 MPa, a temperature of 160-220°C, and a time of 10 minutes to 4 hours.

**11.** The joint treatment method according to any one of claims 1-8, **characterized in that**, in Step 3 or step S3, said separation method comprises:

A. subjecting the reaction product obtained in Step 2 or step S2 to a third rectification at a column top pressure of 20-201 kPa and a column top temperature of 50-160°C, controlling the number of column plates of the third rectification to be 5-42 and the reflux ratio to be 1-15, to separate out a second light component;
B. subjecting the non-distilled material obtained from the third rectification to a fourth rectification under conditions of a column top pressure of 5 Pa - 101 kPa and a column top temperature of 70-170°C, controlling the number of column plates of the fourth rectification to be 5-42 and the reflux ratio to be 1-15, to separate out a material containing glycolates.

**12.** The joint treatment method according to any one of claims 1-11, **characterized in that** said joint treatment method further comprises: returning at least a portion of the residual material after separating out said material containing glycolates to the reaction step to perform reaction.

**13.** The joint treatment method according to claim 8, **characterized in that** said catalyst further comprises 0.1-10 wt% rare earth element oxide; preferably, based on the total weight of the catalyst, said catalyst comprises: 40-70 wt% zinc oxide, 10-40 wt% copper oxide, 5-25 wt% aluminum oxide, and 1-8 wt% rare earth element oxide; preferably, said rare earth element is selected from at least one of lanthanum, cerium, zirconium, and neodymium.

**14.** A system for implementing the joint treatment method according to any one of claims 1-13, comprising: an optional preliminary separation unit, a reaction unit, and an optional product separation unit, wherein said preliminary separation unit is used to at least partially remove water and acid contained in the mixed alcohol waste and optionally separate out a heavy component primarily comprising esters to obtain a preliminary purification product of the mixed alcohol waste; said reaction unit is used to, in the presence of an optional catalyst, allow a mixture comprising heavy alcohol waste, added alcohol, said preliminary purification product of the mixed alcohol waste, and optionally said heavy component primarily comprising esters to react; and said product separation unit is used to separate a material containing glycolates from the reaction product from the reaction unit.

**15.** The system according to claim 14, **characterized in that** said preliminary separation unit comprises a first distillation column and a second distillation column connected in series; said reaction unit comprises one or more reaction kettles; and said product separation unit comprises a third distillation column and a fourth distillation column connected in series.

mixed alcohol waste
preliminary separation unit
first intermediate component
first light component
first heavy component
second light component
heavy alcohol waste
reaction unit
product separation unit
methanol
second intermediate component
second heavy component

FIG.1

mixed alcohol waste
preliminary separation unit
first intermediate component
first light component
second light component
first heavy component
heavy alcohol waste
reaction unit
product separation unit
methanol
second intermediate component
second heavy component

FIG.2

**INTERNATIONAL SEARCH REPORT**

International application No. **PCT/CN2024/122229**

**A. CLASSIFICATION OF SUBJECT MATTER**

C07C67/08(2006.01)i

According to International Patent Classification (IPC) or to both national classification and IPC

**B. FIELDS SEARCHED**

Minimum documentation searched (classification system followed by classification symbols)

IPC： C07C

Documentation searched other than minimum documentation to the extent that such documents are included in the fields searched

Electronic data base consulted during the international search (name of data base and, where practicable, search terms used)

CNTXT, ENTXT, CNKI, STN: 聚乙醇酸, 乙二醇, 乙醇酸甲酯, 醇解, polyglycolic acid, ethylene glycol, methyl glycolate, alcoholysisa

**C. DOCUMENTS CONSIDERED TO BE RELEVANT**

| Category* | Citation of document, with indication, where appropriate, of the relevant passages | Relevant to claim No. |
|---|---|---|
| X | CN 111777508 A (SHANGHAI PUJING CHEMICAL INDUSTRY CO., LTD.) 16 October 2020 (2020-10-16) description, paragraphs 16-22 | 14-15 |
| X | CN 105367386 A (CHINA PETROLEUM & CHEMICAL CORP. et al.) 02 March 2016 (2016-03-02) claim 1 | 14-15 |
| A | CN 107814709 A (JIANGSU JINJU ALLOY MATERIAL CO., LTD.) 20 March 2018 (2018-03-20) entire document | 1-15 |
| A | CN 116623310 A (JIANGSU SAIWEIER NEW MATERIAL TECHNOLOGY CO., LTD.) 22 August 2023 (2023-08-22) entire document | 1-15 |
| A | CN 114656684 A (KOZA NOVEL MATERIALS CO., LTD.) 24 June 2022 (2022-06-24) entire document | 1-15 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 December 2024** | **23 December 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/122229**

**C. DOCUMENTS CONSIDERED TO BE RELEVANT**

| Category* | Citation of document, with indication, where appropriate, of the relevant passages | Relevant to claim No. |
|---|---|---|
| A | CN 111777508 A (SHANGHAI PUJING CHEMICAL INDUSTRY CO., LTD.) 16 October 2020 (2020-10-16)<br>description, paragraphs 16-22 | 1-13 |
| A | CN 105367386 A (CHINA PETROLEUM & CHEMICAL CORP. et al.) 02 March 2016 (2016-03-02)<br>claim 1 | 1-13 |

Form PCT/ISA/210 (second sheet) (July 2022)

**INTERNATIONAL SEARCH REPORT**
**Information on patent family members**

International application No.
**PCT/CN2024/122229**

| Patent document cited in search report | | | Publication date (day/month/year) | Patent family member(s) | Publication date (day/month/year) |
|---|---|---|---|---|---|
| CN | 111777508 | A | 16 October 2020 | None | |
| CN | 105367386 | A | 02 March 2016 | None | |
| CN | 107814709 | A | 20 March 2018 | None | |
| CN | 116623310 | A | 22 August 2023 | None | |
| CN | 114656684 | A | 24 June 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 108250481 A
- CN 111203202 A
- CN 114031600 A